# EUROPEAN PATENT APPLICATION

(11) **EP 4 736 842 A1**
(43) Date of publication of application: **06.05.2026**
(21) Application number: 25212882.2
(22) Date of filing: 03.11.2025
(51) Int. Cl.: A61K 9/00, A61K 31/496, A61K 31/519, A61K 31/7048, A61K 38/26, A61K 47/10, A61K 47/14, A61K 47/20, A61K 47/24, A61K 47/26, A61K 47/34, A61K 47/44, A61K 47/69, A61P 3/10, A61P 25/18

(54) **IN SITU GEL FORMULATION AND PREPARATION METHOD THEREOF**

(30) Priority: 04.11.2024 US 202463715639 P
(71) Applicant: ANXO Pharmaceutical Co., Ltd., Taipei City 104 (TW)
(72) Inventor: LEE, Hsiang-Ju, 104 Taipei City (TW); SU, Chia-Yu, 104 Taipei City (TW); CHEN, Mei-Huei, 104 Taipei City (TW); LIU, Yi-Hsiang, 104 Taipei City (TW); JHAN, Hua-Jing, 104 Taipei City (TW); JIAN, Chi-Heng, 104 Taipei City (TW)
(74) Representative: Berggren Oy

(57) **Abstract**

In some embodiments of the present disclosure, an in situ gel formulation is provided, comprising an active ingredient, solvent, polymer and lipid, in which the polymer comprises polylactic-co-glycolic acid (PLGA), polylactic acid (PLA), or a combination thereof, and the lipid comprises fatty acid, fatty alcohol, glyceride, phospholipid, or a combination thereof. In some embodiments of the present disclosure, a preparation method of an in situ gel formulation is further provided.

## Description

### BACKGROUND

### Field of Invention

The present disclosure relates to in situ gel formulation and preparation method thereof. In particular, the present disclosure relates to in situ gel formulation comprising polymer and lipid.

### Description of Related Art

The in situ gel formulation is a gel implant formulation with sustained-release properties and usually required for high content of polymer. However, high content of polymer has certain drawbacks such as, the use of larger needle sizes due to excessively high viscosity, storage and transportation costs due to requirement for low storage temperature to maintain quality, inflammatory reactions caused by polymer degradation by-products.

Therefore, how to provide an in situ gel formulation to improve the above mentioned problem remains to be solved.

### SUMMARY

In one aspect of the present disclosure, an in situ gel formulation is provided, comprising an active ingredient, solvent, polymer and lipid, in which the polymer comprises PLGA, PLA, or a combination thereof, and the lipid comprises fatty acid, fatty alcohol, glyceride, phospholipid, or a combination thereof.

In some embodiments, a weight percentage of the active ingredient is from 0.1% to 35% based on 100% by weight percentage of the in situ gel formulation.

In some embodiments, the active ingredient comprises a psychiatric drug, or an antidiabetic drug.

In some embodiments, the psychiatric drug comprises cariprazine, brexpiprazole, paliperidone, risperidone, aripiprazole, lurasidone, or a pharmaceutically acceptable salt of any of the foregoing psychiatric drug, or a combination thereof.

In some embodiments, the antidiabetic drug comprises empagliflozin, dapagliflozin, canagliflozin, semaglutide, liraglutide, dulaglutide, tirzepatid, a pharmaceutically acceptable salt of any of the foregoing antidiabetic drug, or a combination thereof.

In some embodiments, a weight percentage of the polymer is from 2.5% to 20% based on 100% by weight percentage of the in situ gel formulation.

In some embodiments, a molar ratio of lactic acid to glycolic acid in the polylactic-co-glycolic acid (PLGA) is from 50:50 to 99:1.

In some embodiments, a weight percentage of the lipid is from 17.5% to 60% based on 100% by weight percentage of the in situ gel formulation.

In some embodiments, a weight percentage of the lipid is from 55% to 95% based on 100% by total weight percentage of the polymer and the lipid.

In some embodiments, a weight percentage of the polymer and the lipid is from 25% to 65% based on 100% by weight percentage of the in situ gel formulation.

In some embodiments, the solvent includes ethanol (EtOH), dimethyl sulfoxide (DMSO), 1-methyl-2-pyrrolidone (NMP), or a combination thereof.

In some embodiments, the in situ gel formulation further comprises a surfactant.

In some embodiments, the surfactant has a hydrophilic-lipophilic balance (HLB) value ranging from 15 to 20.

In some embodiments, a hydrophilic-lipophilic balance (HLB) value of the surfactant is from 16 to 18.

In some embodiments, a weight percentage of the surfactant is from 2.5% to 10% based on 100% by weight percentage of the in situ gel formulation.

In one aspect of the present disclosure, a preparation method of an in situ gel formulation is provided, comprising: mixing an active ingredient, polymer and a first solvent to form a first mixture; mixing lipid and a second solvent to form a second mixture; and mixing the first mixture and the second mixture to form an in situ gel formulation.

In some embodiments, the mixing the active ingredient, the polymer and the first solvent comprises: mixing the active ingredient and the first solvent to form a pre-mixture; and mixing the pre-mixture and the polymer to form the first mixture.

In some embodiments, the mixing the lipid and the second solvent comprises mixing the lipid, the second solvent and a surfactant.

In one aspect of the present disclosure, a preparation method of an in situ gel formulation is provided, comprising: mixing polymer and a solvent to form a polymer mixture; mixing an active ingredient and lipid to form a lipid mixture; and mixing the polymer mixture and the lipid mixture to form an in situ gel formulation.

In some embodiments, the mixing the polymer and the solvent comprises mixing the polymer, the solvent and a surfactant.

### BRIEF DESCRIPTION OF THE DRAWINGS

Fig. 1A illustrates a flow chart of a preparation method of an in situ gel formulation in some embodiments of the present disclosure.
Fig. 1B illustrates a flow chart of another preparation method of an in situ gel formulation in some embodiments of the present disclosure.
Fig. 2 illustrates the release profiles of part (2) of Example 1 (active ingredient: brexpiprazole; the control group: BA12FD) in the in vitro release test.
Fig. 3 illustrates the release profiles of part (2) of Example 2 (active ingredient: cariprazine; the control groups: TF20-752 and TF20-502) in the in vitro release test.
Fig. 4 illustrates the release profiles of part (3) of Example 2 (active ingredient: cariprazine; the control group: TF20-502) in the stability test for one-month storage at room temperature.
Fig. 5 illustrates the release profiles of part (2) of Example 3 (active ingredient: cariprazine) in the in vitro release test.
Fig. 6 illustrates the release profiles of part (2) of Example 4 (active ingredient: dapagliflozin) in the in vitro release test.
Fig. 7 illustrates the release profiles of part (2) of Example 5 (active ingredient: semaglutide) in the in vitro release test.
Fig. 8 illustrates images of in situ gel formulations of part (3) of Example 5 (active ingredient: semaglutide) in the in vivo test in rats.
Fig. 9 illustrates the release profiles of part (2) of Example 6 (active ingredient: semaglutide, paired with different solvent systems) in the in vitro release test.

### DETAILED DESCRIPTION

### Definition

In this description, unless the context specifically dictates otherwise, "a" and "the" may mean a single or a plurality. It will be further understood that "comprise", "include", "have", and similar terms as used herein indicate described features, regions, integers, steps, operations, elements and/or components, but not exclude other features, regions, integers, steps, operations, elements, components and/or groups.

As used herein, "in situ gel formulation" refers to a liquid preparation that undergoes sol-to-gel transition upon exposure to physiological conditions, thereby forming a gel matrix at the site of administration.

As used herein, "active ingredient" refers to specific drug, its esters, its complexes, its chelating agents, its cage compounds, its racemates, its mirror image isomers, or the like.

As used herein, "excipients" refers to pharmaceutical additives without pharmacological activity and used in pharmaceutical compositions according to different purposes and functions.

As used herein, "small molecule drug" refers to a drug having a molecular weight of less than 900 Da.

As used herein, "macromolecule drug" refers to a drug having a molecular weight of greater than (but not including) 900 Da.

### In situ gel formulation and Preparation Method Thereof

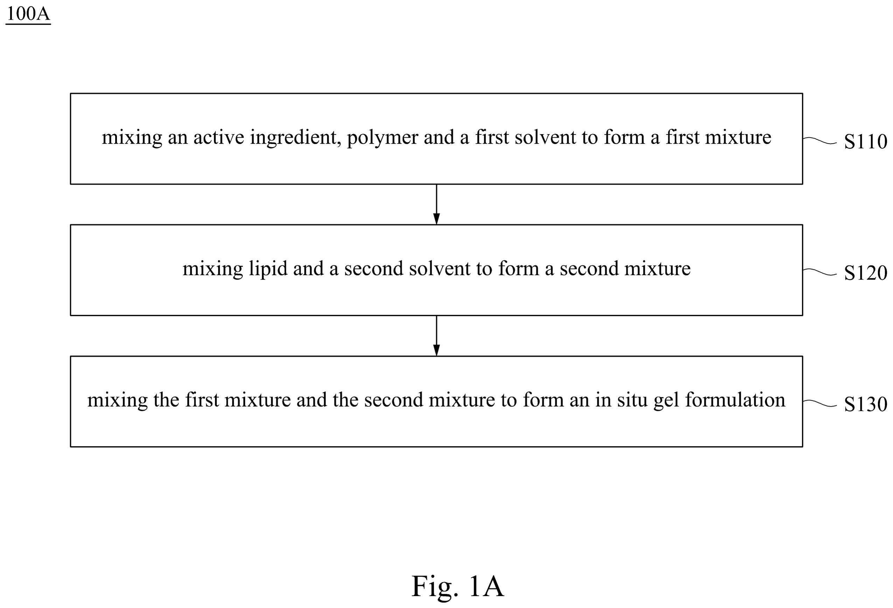

In one aspect of the present disclosure, please refer to Fig. 1A, representing a flow chart of a preparation method 100A of an in situ gel formulation in some embodiments of the present disclosure, comprising step S110, step S120 and step S130.

The in situ gel formulation of the present disclosure reduces viscosity and inflammation by addition of lipid to polymer-containing formulation to reduce polymer content while maintaining a long-acting sustained-release effect. Additionally, it allows for administration using smaller needles, thereby further reducing the degree of inflammation. The condition requiring low-temperature storage in the in situ gel formulation of prior art has also been overcome.

First of all, refer to step S110, mixing an active ingredient, polymer and a first solvent to form a first mixture.

In some embodiments, the active ingredient comprises a psychiatric drug or an antidiabetic drug. The psychiatric drug comprises cariprazine, brexpiprazole, paliperidone, risperidone, aripiprazole, lurasidone, a pharmaceutically acceptable salt of any of the foregoing psychiatric drug, or a combination thereof. The antidiabetic drug comprises a small molecule drug, a macromolecule drug, or a combination thereof. The small molecule drug of the antidiabetic drug comprises empagliflozin, dapagliflozin, canagliflozin, a pharmaceutically acceptable salt of any of the foregoing antidiabetic drug, or a combination thereof. In some embodiments, the macromolecule drug of the antidiabetic drug comprises a glucagon-like peptide-1 (GLP-1) drug, such as semaglutide, liraglutide, dulaglutide, tirzepatide, a pharmaceutically acceptable salt of any of the foregoing GLP-1 drug, or a combination thereof. In some embodiments, the active ingredient comprises a small molecule drug or a macromolecule drug.

In some embodiments, the polymer comprises polylactic-co-glycolic acid (PLGA), polylactic acid (PLA), or a combination thereof. In some embodiments, a molar ratio of lactic acid to glycolic acid in the PLGA is from 50:50 to 99:1, such as 50:50, 65:35, 75:25, 85:15, 95:5, 99:1, or a value within the interval defined between any two of the foregoing numerical values.

In some embodiments, the first solvent comprises ethanol (EtOH), dimethyl sulfoxide (DMSO), 1-methyl-2-pyrrolidone (NMP), or a combination thereof. It is noted that compared with ethanol, using DMSO or NMP as the first solvent can achieve better solubility of polymer in the solvent. In some embodiments, the first solvent comprises DMSO, NMP, or a combination thereof.

In some embodiments, the mixing the active ingredient, the polymer and the first solvent comprises: mixing the active ingredient and the first solvent to form a pre-mixture; and mixing the pre-mixture and the polymer to form the first mixture. It's noted that mixing the active ingredient in the first solvent before mixing with the polymer ensures that the active ingredient is previously dissolved or suspended in the first solvent, thereby increasing uniformity of the first mixture.

In some embodiments, the lipid comprises fatty acid, fatty alcohol, glyceride, phospholipid, or a combination thereof.

In some embodiments, the fatty acid comprises medium- and long-chain fatty acids, such as caprylic acid, capric acid, lauric acid, myristic acid, palmitic acid, stearic acid, oleic acid, arachidic acid, behenic acid, or lignoceric acid.

In some embodiments, the fatty alcohol comprises medium- and long-chain fatty alcohols, such as capryl alcohol, capric alcohol, lauryl alcohol, myristyl alcohol, palmitoleyl alcohol, stearyl alcohol, oleyl alcohol, arachidyl alcohol, behenyl alcohol, or lignoceryl alcohol.

In some embodiments, the glyceride comprises mono-glyceride, di-glycerides, triglycerides, or a combination thereof. In some embodiments, the glyceride comprises medium-chain glycerides, long-chain glycerides, or a combination thereof. In some embodiments, medium- and long-chain mono- and di-glycerides are Glycerol Monocaprylocaprate (IMWITOR^{®}-742), Glyceryl Monostearate (IMWITOR^{®} 491), Glycerol Monostearate 40%-55%, type I (IMWITOR^{®} 900K), Glyceryol Mono Oleate (IMWITOR^{®} 948), Glycerol monocaprylate, type I (IMWITOR^{®} 988), or a combination thereof; medium- and long-chain triglycerides are medium-chain triglycerides (MIGLYOL^{®}-810N or MIGLYOL^{®}-812N), Caprylic/Capric/Succinic Triglyceride (MIGLYOL^{®}-829), Propylene Glycol Dicaprylocaprate (MIGLYOL^{®}-840), tricaprylin, tricaprin, trilaurin, trimyristin, tripalmitin, tristearin, triolein, soybean oil, coconut oil, castor oil, or a combination thereof.

In some embodiments, the phospholipid comprises 1,2-Dipalmitoyl-sn-glycero-3-phosphocholine (DPPC), 1,2-Distearoyl-sn-glycero-3-phosphocholine (DSPC), 1,2-Dioleoyl-sn-glycero-3-phosphocholine (DOPC), 1,2-Diarachidoyl-sn-glycero-3-phosphocholine (DAPC), or a combination thereof. In some embodiments, the phospholipid comprises phosphatidylcholine derived from soybean, such as LIPOID S100 or LIPOID SPC.

In some embodiments, the mixing the lipid and the second solvent comprises mixing the lipid, the second solvent and a surfactant. By adding the surfactant, mixing uniformity of the lipid and the second solvent is enhanced. In some embodiments, the surfactant has a hydrophilic-lipophilic balance (HLB) value ranging from 15 to 20, such as from 16 to 18, for further increasing mixing uniformity of the first mixture. In some embodiments, the surfactant includes polysorbate 20, polysorbate 40, polysorbate 60, polysorbate 80, polyoxyethylene (23) lauryl ether (Brij 35), sodium oleate, potassium oleate, or a combination hereof.

Furthermore, refer to step S120, mixing lipid and a second solvent to form a second mixture. It's noted that the design that dissolving the polymer in the first solvent and dissolving the lipid in the second solvent respectively ensures the polymer and the lipid are respectively dissolved uniformly, thereby enhancing the uniformity of the solute in the in situ gel formulation in the following mixing step.

In some embodiments, the second solvent comprises EtOH, DMSO, NMP, or a combination thereof. It is noted that compared with DMSO or NMP, using EtOH as the second solvent can achieve better solubility of lipid. In some embodiments, the second solvent comprises EtOH.

Furthermore, refer to step S130, mixing the first mixture and the second mixture to form an in situ gel formulation.

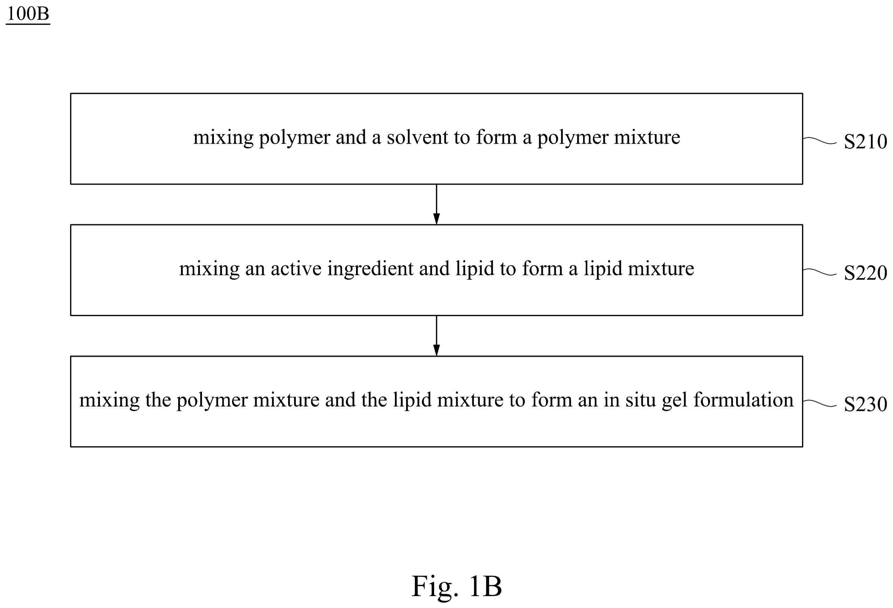

In another aspect of the present disclosure, please refer to Fig. 1B, representing a flow chart of a preparation method 100B of an in situ gel formulation in some embodiments of the present disclosure, comprising step S210, step S220 and step S230, in which step S210, step S220 and step S230 respectively represent mixing polymer and a solvent to form a polymer mixture (step S210), mixing an active ingredient and lipid to form a lipid mixture (step S220) and mixing the polymer mixture and the lipid mixture to form an in situ gel formulation (step S230).

The materials used in the preparation method 100B are basically similar to those in the preparation method 100A (Fig. 1A), and the solvent in the preparation method 100B is similar to the first solvent or the second solvent in the preparation method 100A (Fig. 1A). The primary difference lies in the fact that, in preparation method 100B, the active ingredient is first mixed with the lipid instead of the polymer.

In some embodiments, mixing the polymer and the solvent (step S210) comprises mixing the polymer, the solvent and a surfactant. That is, in preparation method 100B, the surfactant is first mixed with the polymer instead of the lipid.

In some embodiments, an in situ gel formulation is provided according to the abovementioned preparation method 100A (Fig. 1A) or preparation method 100B (Fig. 1B), comprising an active ingredient, solvent, polymer and lipid, in which the polymer comprises PLGA, PLA, or a combination thereof, and the lipid comprises fatty acid, fatty alcohol, glyceride, phospholipid, or a combination thereof. It is understood that people skill in the art can select either the preparation method 100A or preparation method 100B according to practical demand to obtain an in situ gel formulation, and the in situ gel formulation prepared by either of preparation method 100A (Fig. 1A) or preparation method 100B (Fig. 1B) can achieve efficiency of viscosity and inflammation reduction while maintaining a long-acting sustained-release effect.

It should be noted that, since the required percentage of polymer in the in situ gel formulation can be reduced by addition of lipid, viscosity of the in situ gel formulation can be reduced, which makes the in situ gel formulation allows for administration using smaller needles and the wider storage temperature, and the extent of inflammation in surrounding biological tissues caused by polymer degradation by-products can be reduced. For example, compared with the gel formulations of prior art, which requires the use of larger gauge needles (approximately 18G-20G) for administration since viscosity is relatively high, the in situ gel formulation of the present invention can be administered using smaller gauge needles (approximately 23G-26G) due to the lower viscosity.

Additionally, the in situ gel formulation disclosed in the present disclosure exhibits improved stability compared with prior art. Unlike the gel formulation in the prior art, which shows an significant increasing or decreasing trend in active drug release after one month of storage at room temperature, the in situ gel formulation of the present disclosure maintains a similar in vitro drug release profile to its initial state even after at least one month of storage at room temperature (e.g., 25°C to 30°C). Furthermore, it is worth noting that the in situ gel formulation represents maintained good structural integrity even after 28 days of implantation in vivo, suitable for prolonged support or drug release.

On the other hand, relative to a gel formulation comprising lipid alone, the present in situ gel formulation, owing to the reinforcement provided by the polymer, is capable of achieving an enhanced sustained-release effect.

In some embodiments, the solvent (the first solvent and the second solvent) comprises ethanol (EtOH), dimethyl sulfoxide (DMSO), 1-methyl-2-pyrrolidone (NMP), or a combination thereof.

In some embodiments, a weight percentage of the active ingredient is from 0.1% to 35% based on 100% by weight percentage of the in situ gel formulation, such as 0.1%, 1%, 5%, 10%, 15%, 20%, 25%, 30%, 35%, or a value within the interval defined between any two of the foregoing numerical values. By maintaining the weight percentage of the active ingredient within a suitable range, the desired content of the active ingredient is ensured while burst release of the active ingredient is prevented.

In some embodiments, a weight percentage of the polymer is from 2.5% to 20% based on 100% by weight percentage of the in situ gel formulation, such as 2.5%, 5%, 10%, 15%, 20%, or a value within the interval defined between any two of the foregoing numerical values. In some embodiments, a weight percentage of the lipid is from 17.5% to 60% based on 100% by weight percentage of the in situ gel formulation, such as 17.5%, 20%, 25%, 30%, 35%, 40%, 45%, 50%, 55%, 60%, or a value within the interval defined between any two of the foregoing numerical values. By maintaining the weight percentage of the polymer and the lipid within a suitable range, the efficacy of gel formation and long-acting sustained-release are ensured.

In some embodiments, a weight percentage of the lipid is from 55% to 95% based on 100% by total weight percentage of the polymer and the lipid, such as 55%, 60%, 65%, 70%, 75%, 80%, 85%, 90%, 95%, or a value within the interval defined between any two of the foregoing numerical values. By maintaining the weight percentage of the lipid in the polymer and the lipid within a suitable range, the efficacy of gel formation and long-acting sustained-release are ensured.

In some embodiments, a weight percentage of the polymer and the lipid is from 25% to 65% based on 100% by weight percentage of the in situ gel formulation, such as 25%, 30%, 35%, 40%, 45%, 50%, 55%, 60%, 65%, or a value within the interval defined between any two of the foregoing numerical values. By maintaining the weight percentage of the polymer and the lipid in the in situ gel formulation within a suitable range, the efficacy of gel formation and long-acting sustained-release are ensured.

In some embodiments, the in situ gel formulation further comprises a surfactant. In some embodiments, a weight percentage of the surfactant is from 2.5% to 10% based on 100% by weight percentage of the in situ gel formulation, such as 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, or a value within the interval defined between any two of the foregoing numerical values. By controlling the surfactant within a suitable range, the homogeneity of the in situ gel formulation can be improved, while avoiding potential issues arising from excessive surfactant, such as formulation instability, biocompatibility concerns, and micellization-induced interference with release.

In some embodiments, when the active ingredient comprises cariprazine, the dissolution percentage of the in situ gel formulation in a release buffer of pH 5.5 including acetate is from 10% to 50% (such as, 10%, 20%, 30%, 40%, 50%, or a value within the interval defined between any two of the foregoing numerical values) at day 14 and from 10% to 85% (such as, 10%, 20%, 30%, 40%, 50%, 60%, 70%, 80%, 85%, or a value within the interval defined between any two of the foregoing numerical values) at day 28.

In some embodiments, when the active ingredient comprises dapagliflozin, the dissolution percentage of the in situ gel formulation in a release buffer of pH 7.0 including phosphate is from 10% to 35% (such as, 10%, 20%, 30%, 35%, or a value within the interval defined between any two of the foregoing numerical values) at day 14 and from 10% to 40% (such as, 10%, 20%, 30%, 40%, or a value within the interval defined between any two of the foregoing numerical values) at day 28.

In some embodiments, when the active ingredient comprises semaglutide, the dissolution percentage of the in situ gel formulation in a release buffer of pH 6.8 including phosphate is from 25% to 95% (such as, 25%, 30%, 40%, 50%, 60%, 70%, 80%, 90%, 95%, or a value within the interval defined between any two of the foregoing numerical values) at day 1 and from 1% to 20% (such as, 1%, 5%, 10%, 15%, 20%, or a value within the interval defined between any two of the foregoing numerical values) at day 14.

### Pharmaceutical Composition

A pharmaceutical composition comprising the above mentioned in situ gel formulation is also provided in some embodiments of the present disclosure.

In some embodiments, the pharmaceutical composition comprises at least an excipient, such as thermo-sensitive block copolymers, semi-synthetic cellulose derivatives, polyacrylic acid derivatives, cationic polysaccharides, ion-sensitive polysaccharides, biodegradable polyesters, or a combination thereof.

It should be understood that the above-described embodiments and the following examples are given by way of illustration, not limitation. Various changes and modifications within the scope of the present invention will become apparent to those skilled in the art from the present description.

### Example 1 (Small-Molecule Psychiatric Drug, Brexpiprazole)

### (1) Preparation Method of In Situ Gel Formulation

### Type I (formulas BA12FD, S-6, S-7, O-9-3 and O-9-5)

(a-1) According to the formula disclosed in Table 1, the active ingredient (brexpiprazole), and the first solvent (DMSO) were weighed. Then, the active ingredient was uniformly dispersed in the first solvent using an ultrasonic water bath. The polymer was subsequently added and stirred until it dissolved to obtain a first mixture.

(b-1) The lipid and the second solvent (anhydrous ethanol) were weighed, The lipid were dissolved in the second solvent through stirring to obtain a second mixture.

(c-1) The first mixture and the second mixture prepared in steps (a-1) and (b-1) were mixed and stirred thoroughly to form the in situ gel formulation, with BA12FD serving as the control group.

### Type II (formula O-7-1-1)

(a-2) For formulation O-7-1-1, the polymer, surfactant and the first solvent (DMSO) were weighed according to the formula disclosed in Table 1. Then, the polymer and surfactant dissolved in the solvent as polymer mixture.

(b-2) The active ingredient (brexpiprazole) and lipid were weighed. Then, the active ingredient was uniformly dispersed in lipid using an ultrasonic water bath as lipid mixture.

(c-2) The polymer mixture and the lipid mixture were mixed and stirred thoroughly to form the in situ gel formulation.

It is understood that, since the solvents suitable for polymers and lipids were usually different, in the example the design in which the active ingredient was mixed with the polymer or the lipid, and two mixtures respectively including polymer or lipid were formed, followed by mixing the two mixtures, ensured that the polymer and the lipid were respectively dissolved uniformly, thereby enhancing the uniformity of the solute in the in situ gel formulation.

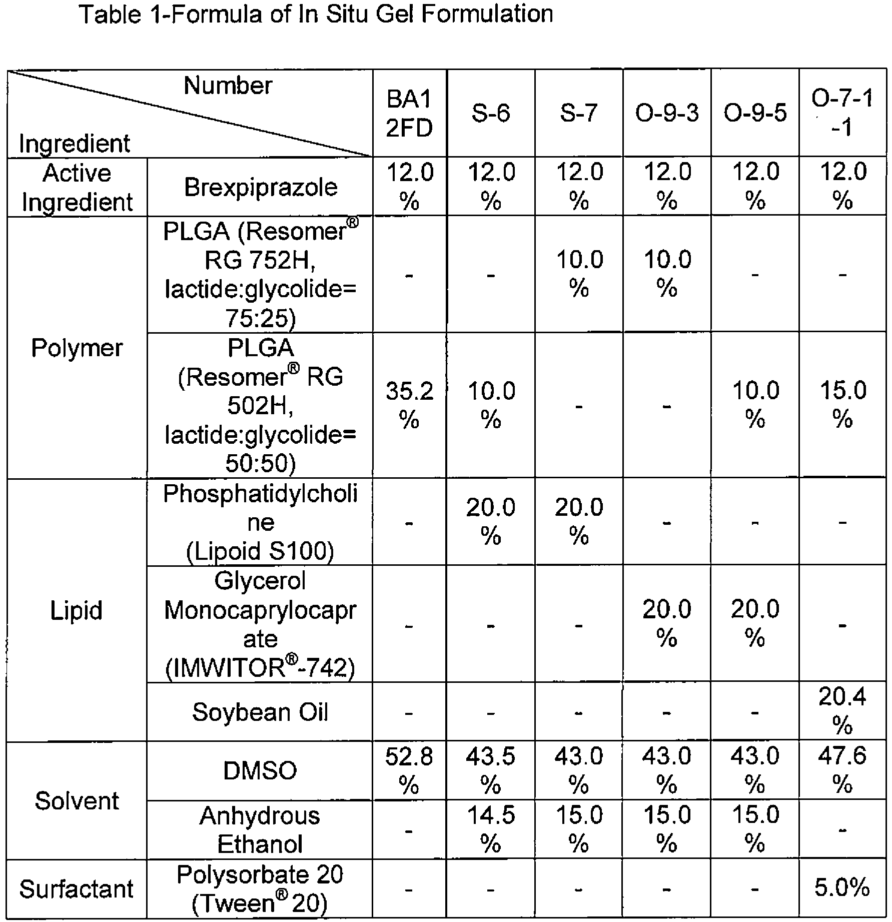

The formulas prepared according to Table 1 were all able to form gels successfully.

### (2) In Vitro Release Test of In Situ Gel Formulation

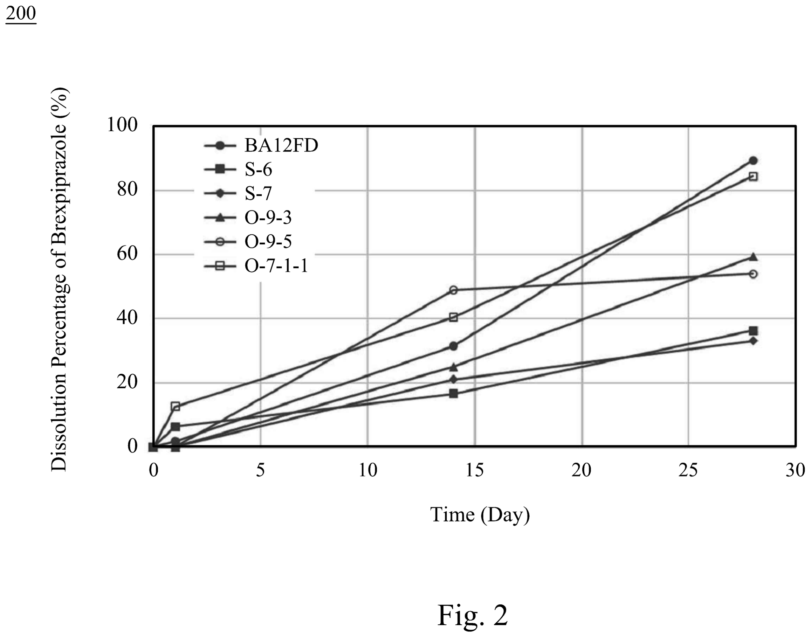

Using a shaker at 100 rpm, an in vitro release test was conducted under the dissolution condition of 10 mL of pH 5.5 acetate buffer (0.05M) for the control group BA12FD and the formulas S-6, S-7, O-9-3, 0-9-5, and O-7-1-1. The result was presented in Table 2 and Fig. 2 (result figure 200).

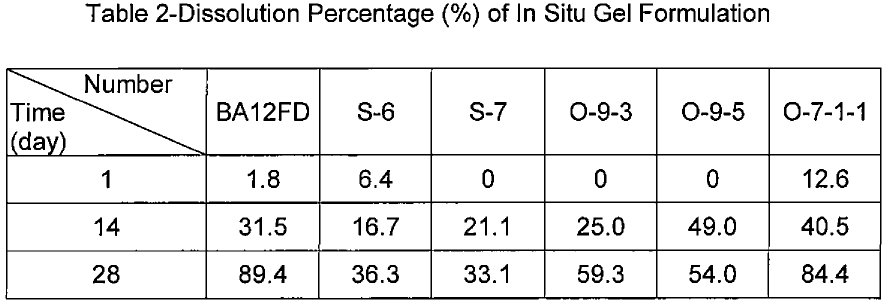

As shown in Table 2 and Fig. 2, the release percentages of the in situ gel formulations disclosed in Example 1 were slower than the control group BA12FD on the 28^{th} day, demonstrating relatively better sustained-release properties. That is, the in situ gel formulations disclosed in Example 1 all formed drug reservoirs.

### Example 2 (Small-Molecule Psychiatric Drug, Cariprazine)

### (1) Preparation Method of In Situ Gel Formulation

(a) According to the formula disclosed in Table 3, the active ingredient (cariprazine), the polymer, and the first solvent (DMSO or NMP) were weighed. Then, the active ingredient was uniformly dispersed in the first solvent using an ultrasonic water bath. The polymer was subsequently added and stirred until it dissolved to obtain a first mixture.
(b) The lipid and the second solvent (anhydrous ethanol) were weighed, and optionally, the surfactant was also weighed (used for formulation C-8), in which the HLB of the surfactant was from 16 to 18 for increasing the mixing of the oil phase and the aqueous phase. The lipid and the surfactant were dissolved in the second solvent through stirring to obtain a second mixture.
(c) The first mixture and the second mixture prepared in steps (a) and (b) were mixed and stirred thoroughly to form the in situ gel formulation, with TF20-752 and TF20-502 serving as the control group.

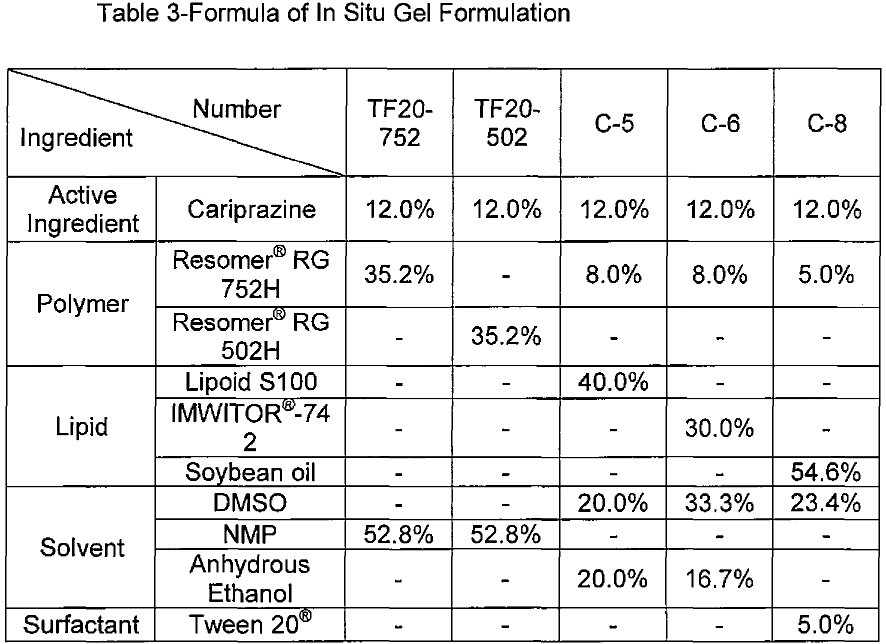

The formulas prepared according to Table 3 were all able to form gels successfully.

### (2) In Vitro Release Test of In Situ Gel Formulation

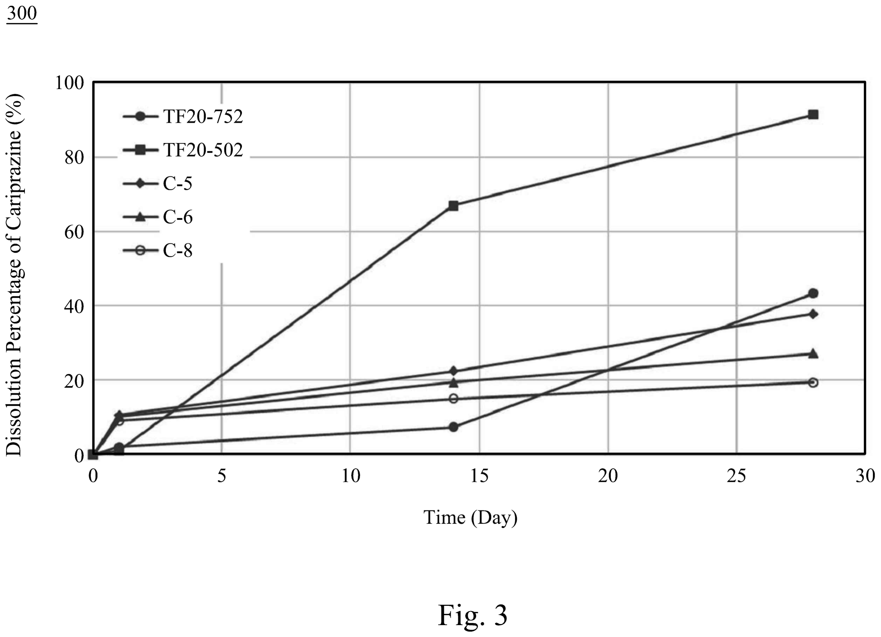

An in vitro release test was conducted basically by the procedure of the in vitro release test similar to Part (2) of Example 1 for the control groups TF20-752 and TF20-502 and the formulas C-5, C-6 and C-8. The result was presented in Table 4 and Fig. 3 (result figure 300).

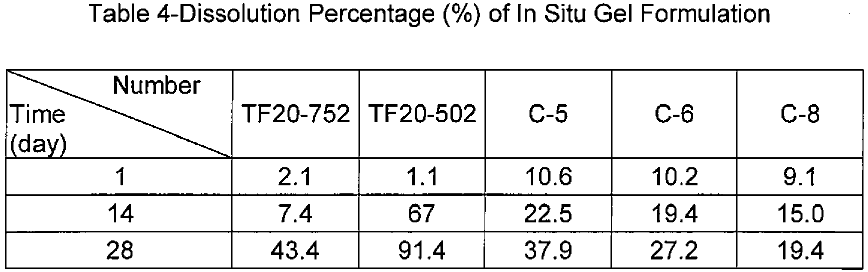

As shown in Table 4 and Fig. 3, the release percentages of the in situ gel formulations disclosed in Example 2 were slower than the control groups TF20-752 and TF20-502 on the 28^{th} day, demonstrating relatively better sustained-release properties. That is, the in situ gel formulations disclosed in Example 2 all formed drug reservoirs.

### (3) Stability Test of In Situ Gel Formulation

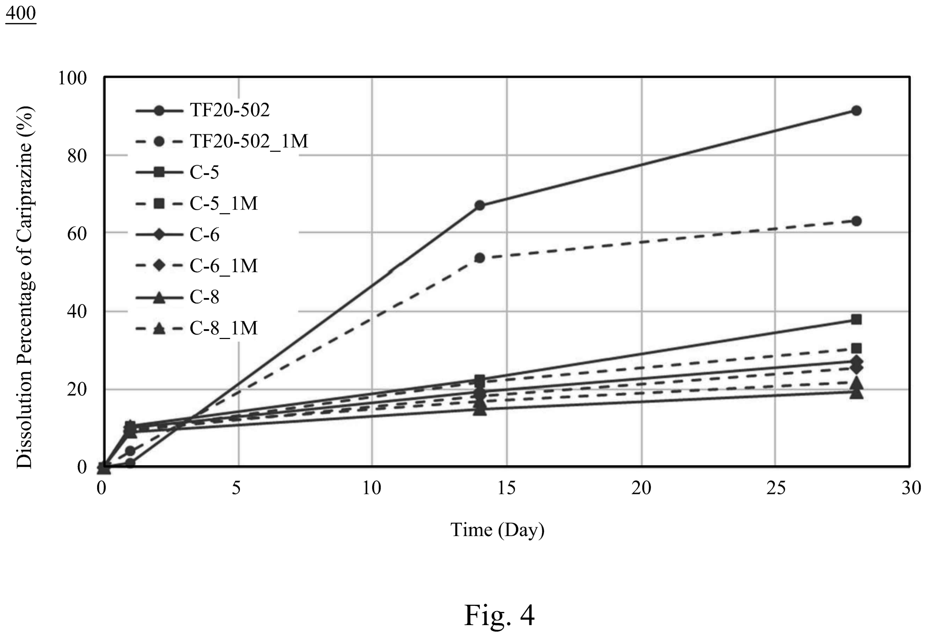

The in situ gel formulations C-5, C-6, and C-8 prepared in part (1) of Example 2, along with the control group TF20-502, were stored in glass sample bottles at room temperature (approximately 25°C-30°C) for one month. Subsequently, an in vitro release test was conducted basically by the procedure of the in vitro release test similar to Part (2) of Example 1. The result was shown in Table 5 and Fig. 4 (result figure 400).

**Table 5- Dissolution Percentage (%) of In Situ Gel Formulation after One Month Storage**

| | Dissolution Percentage (%) | | | | | | | |
|---|---|---|---|---|---|---|---|---|
| | TF20-502 | | C-5 | | C-6 | | C-8 | |
| Time (Day) | Initial State | One Month | Initial State | One Month | Initial State | One Month | Initial State | One Month |
| 1 | 1.1 | 4.2 | 10.6 | 9.4 | 10.2 | 9.4 | 9.1 | 10.4 |
| 14 | 67 | 53.5 | 22.5 | 21.9 | 19.4 | 18.3 | 15.0 | 17.0 |
| 28 | 91.4 | 63 | 37.9 | 30.5 | 27.2 | 25.5 | 19.4 | 21.9 |

As seen in Table 5 and Fig. 4, the dissolution percentage of TF20-502 in the control group decreased by 28% after one month at room temperature, but the dissolution percentages of formulas C-5, C-6 and C-8 did not change significantly, indicating that the in situ gel formulations disclosed in Example 2 remained stable even when stored at room temperature.

### Example 3 (Small-Molecule Psychiatric Drug, Cariprazine)- Different Concentrations of Active Ingredients

### (1) Preparation Method of In Situ Gel Formulation

The in situ gel formulations were prepared according to the formula of Table 6 basically by the preparation method similar to Type I method [step (a-1) to step (c-1)] in Part (1) of Example 1, in which the first solvent was DMSO, and the second solvent was anhydrous ethanol.

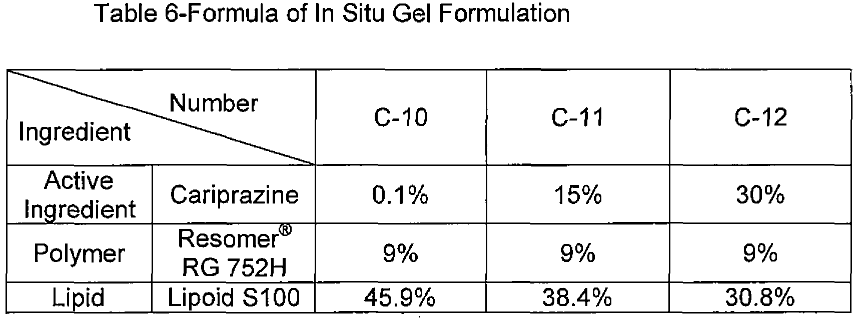

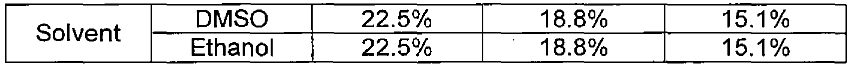

The formulas prepared according to Table 6 were all able to form gels successfully.

### (2) In Vitro Release Test of In Situ Gel Formulation

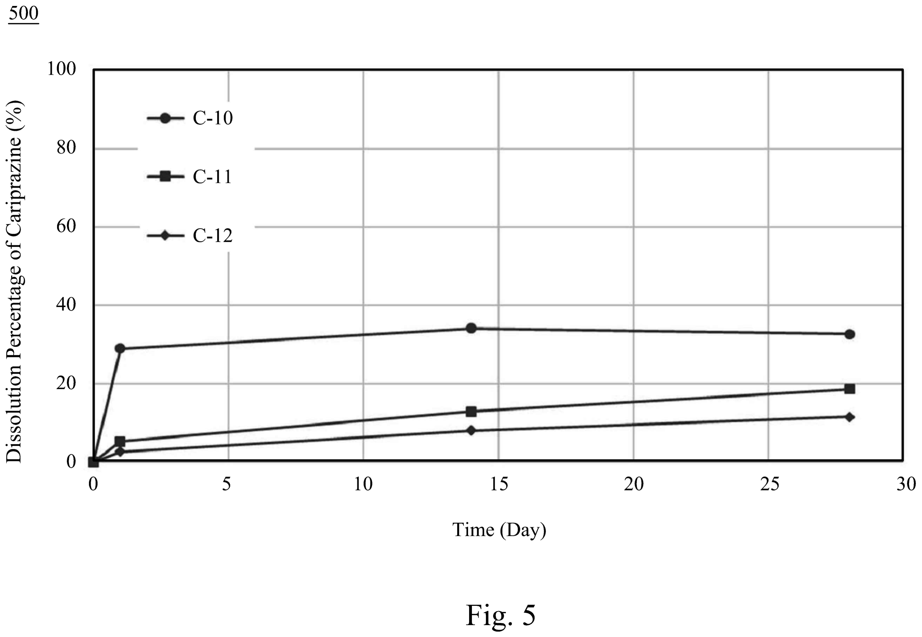

An in vitro release test was conducted basically by the procedure of the in vitro release test similar to Part (2) of Example 1 for the formulas C-10, C-11 and C-12. The result was presented in Table 7 and Fig. 5 (result figure 500).

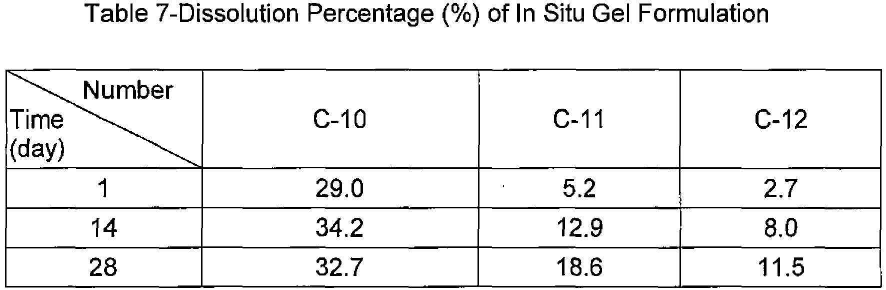

The in situ gel formulations disclosed in Example 3 exhibited excellent drug-loading capacity, capable of carrying drug (active ingredient) concentrations ranging from 0.1% to 30%. The in vitro dissolution tests for different drug concentrations all demonstrated good long-acting sustained-release capabilities.

### Example 4 (Small-Molecule Antidiabetic Drug, Dapagliflozin)

### (1) Preparation Method of In Situ Gel Formulation

The in situ gel formulations were prepared according to the formula of Table 8 basically by the preparation method similar to Type I method [step (a-1) to step (c-1)] in Part (1) of Example 1, in which the first solvent was DMSO, and the second solvent was anhydrous ethanol.

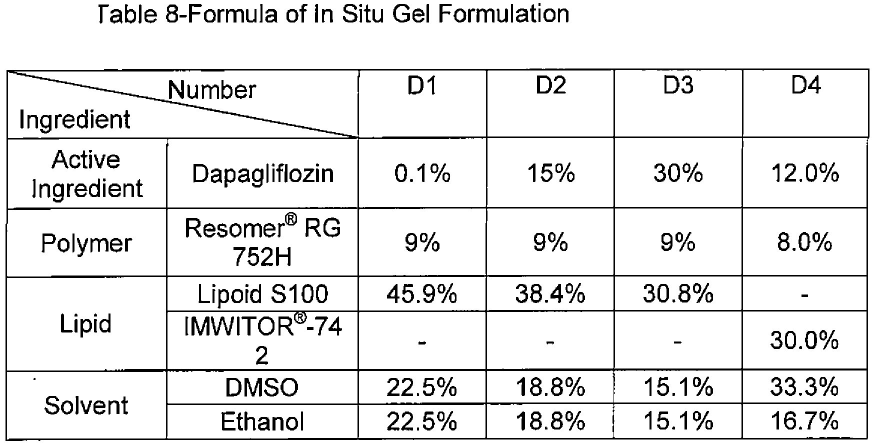

The formulas prepared according to Table 8 were all able to form gels successfully.

### (2) In Vitro Release Test of In Situ Gel Formulation

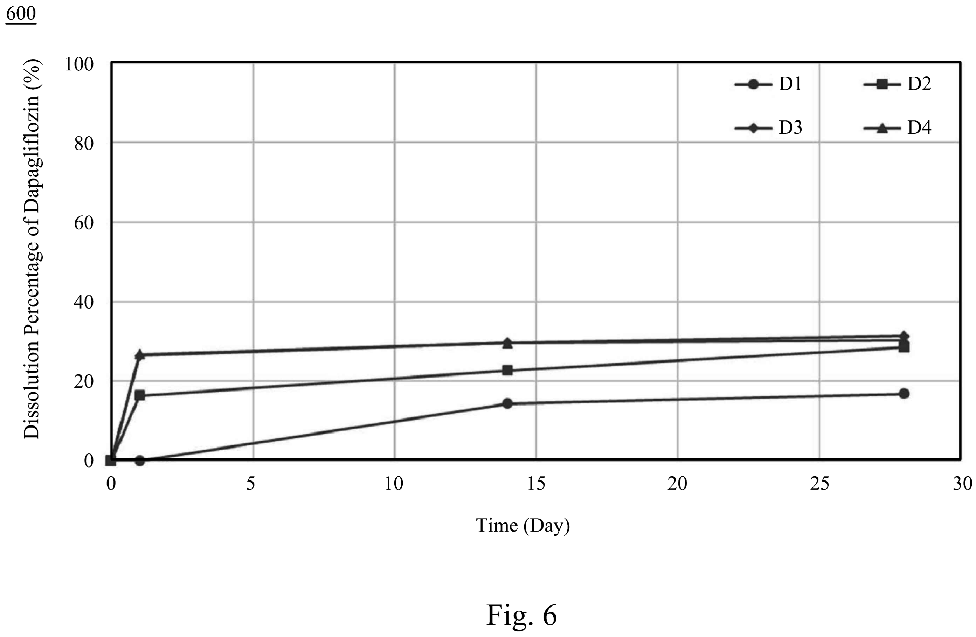

Using a shaker at 100 rpm, an in vitro release test was conducted under the dissolution condition of 10 mL of pH 7.0 phosphate buffer (0.2M) for the formulas D1, D2, D3, and D4. The result was presented in Table 9 and Fig. 6 (result figure 600).

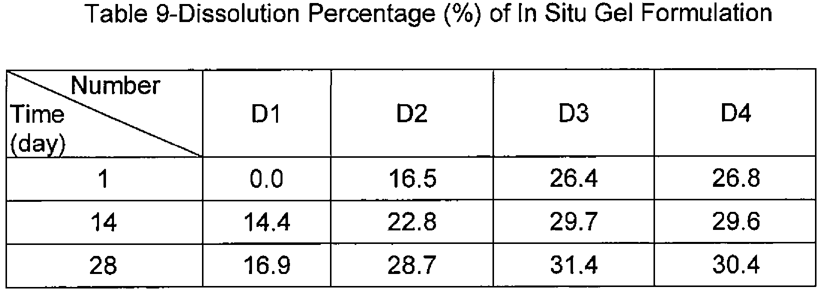

As shown in Table 9 and Fig. 6, the in situ gel formulations D1, D2, and D3 disclosed in the Example once again demonstrated excellent drug-loading capacity and sustained-release properties. Moreover, even when different lipid was used in formula D4, similar effects of drug-loading capacity and sustained-release properties were also achieved.

### Example 5 (Macromolecular Antidiabetic Drug, Semaglutide)

### (1) Preparation Method of In Situ Gel Formulation

The in situ gel formulations were prepared according to the formula of Table 10 basically by the preparation method similar to Type I method [step (a-1) to step (c-1)] in Part (1) of Example 1, in which the first solvent was DMSO, and the second solvent was anhydrous ethanol.

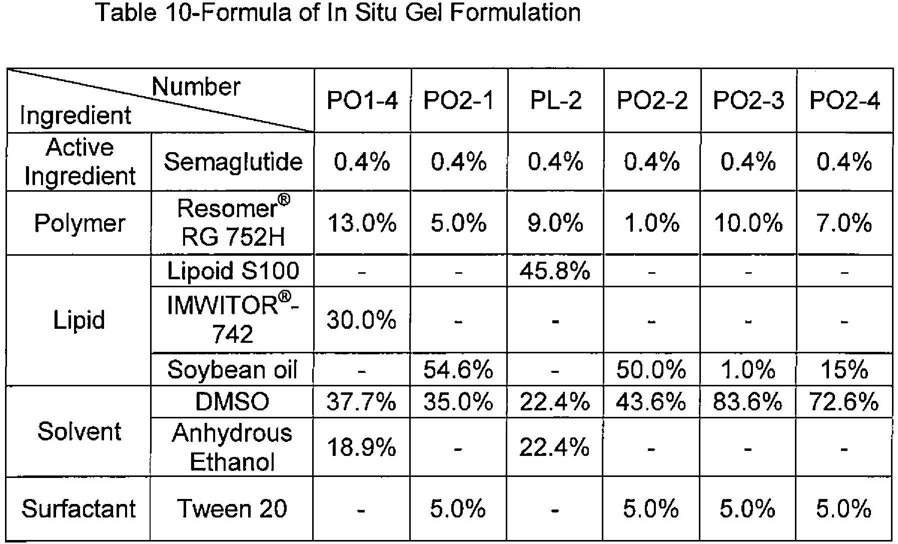

It was observed that the formulas PO2-2, PO2-3, and PO2-4 prepared according to Table 10 were not able to form gels, while the other formulas were able to form gels successfully. Therefore, it was noted that the total percentage of lipid and polymer, as well as the percentage of either the lipid or the polymer in the in situ gel formulation, was controlled within an appropriate range so as to enable successful gel formation.

### (2) In Vitro Release Test of In Situ Gel Formulation

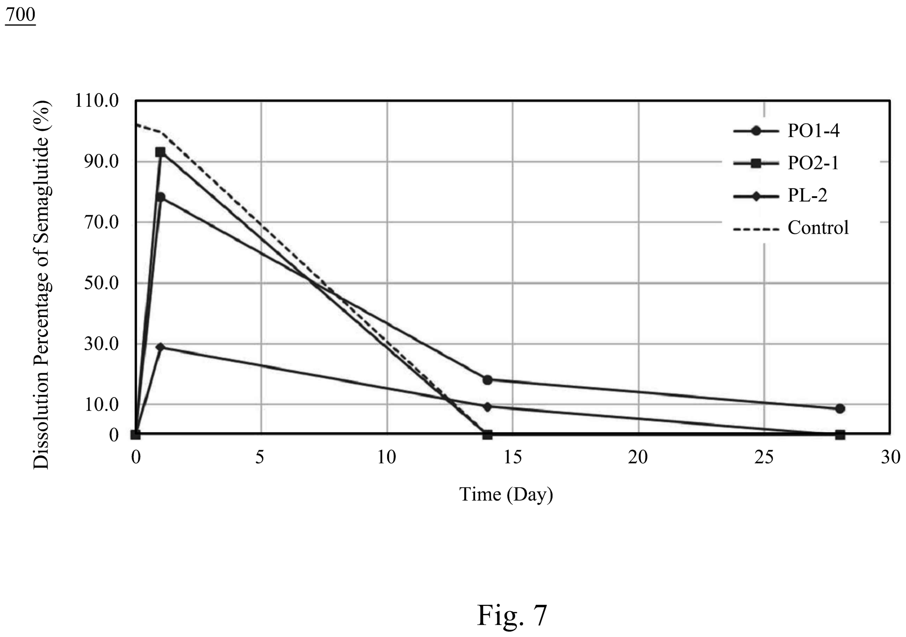

Using a shaker at 100 rpm, an in vitro release test was conducted under the dissolution condition of 10 mL of pH 6.8 phosphate buffer (0.05M) for the formulas PO1-4, PO2-1 and PL-2, and pH 6.8 phosphate buffer in which the active ingredient was dissolved in (also referred to as the active ingredient solution) was used as the control group for stability test of the active ingredient. The result was presented in Table 11 and Fig. 7 (result figure 700).

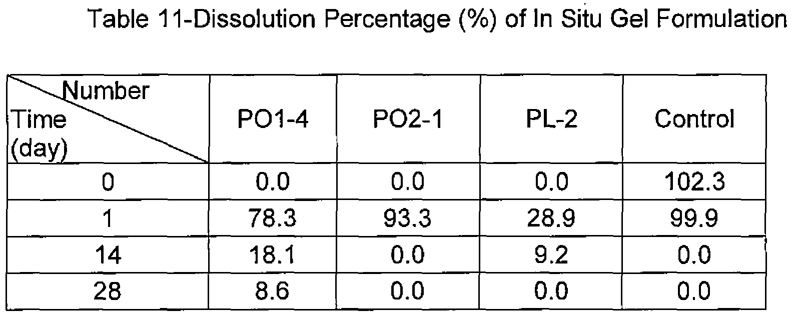

The results, as shown in Table 11 and Fig. 7, indicated that the control group (the active ingredient solution) exhibited poor stability, completely degrading by day 14. In contrast, the in situ gel formulations disclosed in Example 5 demonstrated a stabilizing effect on the active ingredient, each exhibiting different sustained-release properties. Formula PO1-4 showed superior ability to stabilize the active ingredient, while PL-2 exhibited better suppression of drug burst release.

### (3) In Vivo Test in Rat

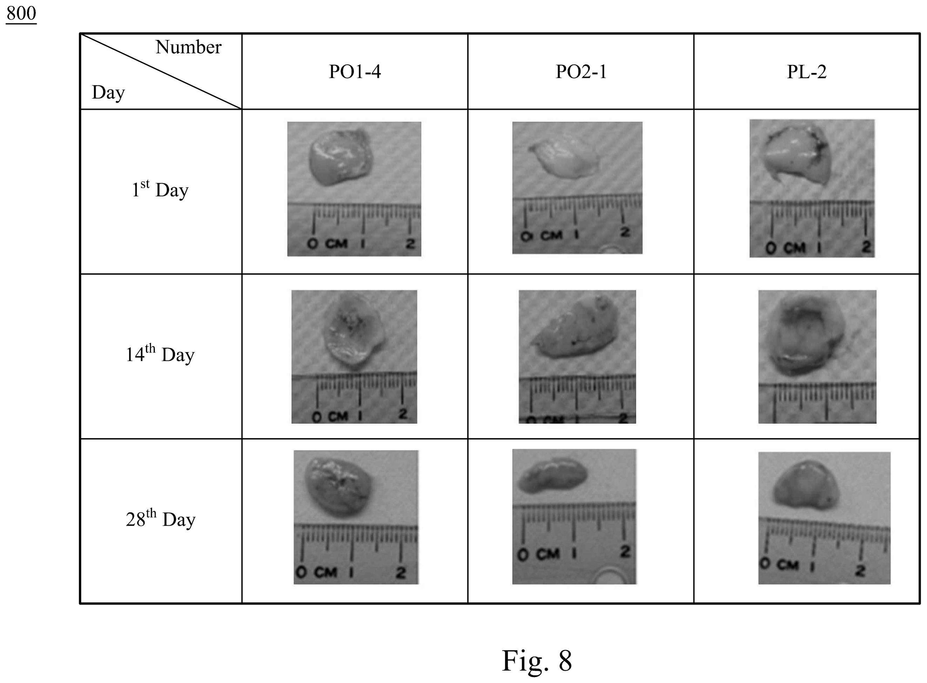

Male Sprague-Dawley (SD) rats were used as the animal model, and subcutaneous injection was performed using a 26G needle. Different groups of in situ gel formulations were administered, with an injection dose of 4 mg/kg and an injection volume of 1 mL/kg for each group. The rats were sacrificed on Day 1, Day 14, and Day 28 post-administration. Subcutaneous implants (in situ gel formulations) were dissected and removed, and the appearance and size of the implants were recorded.as shown in Fig. 8 (result figure 800).

As shown in Fig. 8, the in situ gel formulations disclosed in Example 5 maintained structural integrity even after 28 days of implantation in vivo. No significant degradation or damage was observed, fully demonstrating excellent and durable long-lasting performance of the in situ gel formulations, making the in situ gel formulations suitable for applications requiring prolonged support or drug release.

### Example 6 (Macromolecular Antidiabetic Drug, Semaglutide)-Different Solvents

### (1) Preparation Method of In Situ Gel Formulation

The in situ gel formulations were prepared according to the formula of Table 12 basically by the preparation method similar to Type I method [step (a-1) to step (c-1)] in Part (1) of Example 1, in which the first solvent was DMSO or NMP, and the second solvent was anhydrous ethanol.

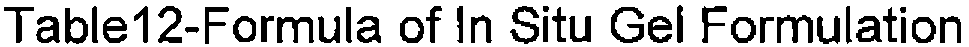

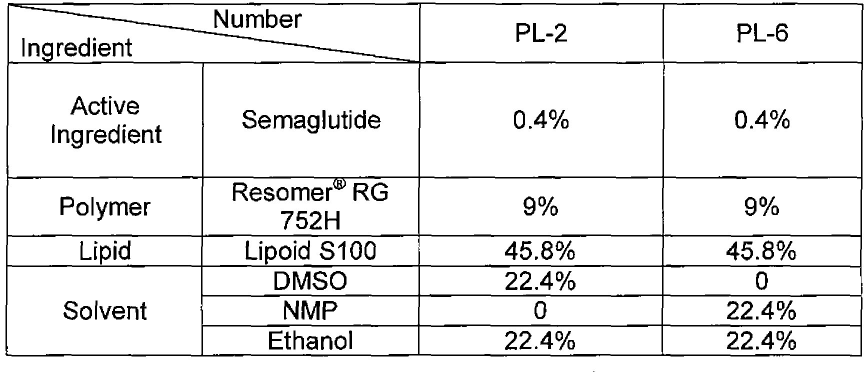

The formulas prepared according to Table 12 were all able to form gels successfully.

### (2) In Vitro Release Test of In Situ Gel Formulation

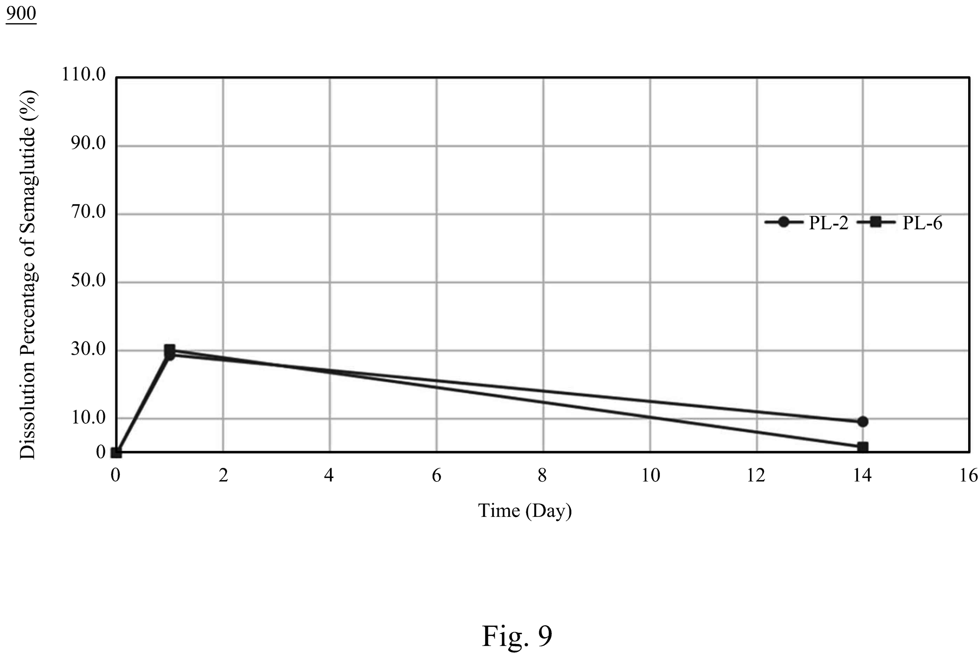

An in vitro release test was conducted basically by the procedure of the in vitro release test similar to Part (2) of Example 5 for the formulas PL-2 and PL-6. The result was presented in Table 13 and Fig. 9 (result figure 900).

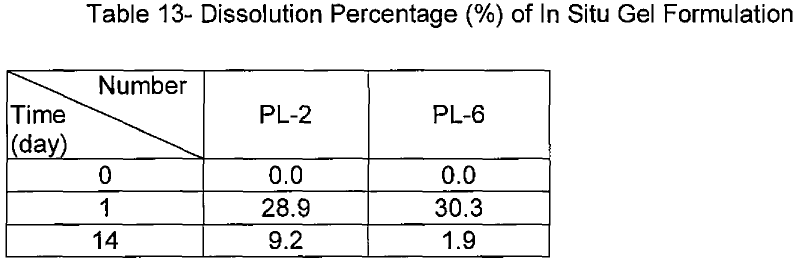

Please refer to Table 13 and Fig. 9. The in situ gel formulations were able to be paired with different solvent systems, showing little differences in in vitro dissolution.

## Claims

1. An in situ gel formulation, **characterized by** comprising:
an active ingredient;
a solvent;
polymer, wherein the polymer comprises polylactic-co-glycolic acid, polylactic acid, or a combination thereof; and
lipid, wherein the lipid comprises fatty acid, fatty alcohol, glyceride, phospholipid, or a combination thereof.

2. The in situ gel formulation of claim 1, **characterized in that** a weight percentage of the active ingredient is from 0.1% to 35% based on 100% by weight percentage of the in situ gel formulation.

3. The in situ gel formulation of any one of claims 1 to 2, **characterized in that** the active ingredient comprises a psychiatric drug or an antidiabetic drug.

4. The in situ gel formulation of claim 3, **characterized in that** the psychiatric drug comprises cariprazine, brexpiprazole, paliperidone, risperidone, aripiprazole, lurasidone, or a pharmaceutically acceptable salt of any of the foregoing psychiatric drug, or a combination thereof, wherein the antidiabetic drug comprises empagliflozin, dapagliflozin, canagliflozin, semaglutide, liraglutide, dulaglutide, tirzepatid, a pharmaceutically acceptable salt of any of the foregoing antidiabetic drug, or a combination thereof.

5. The in situ gel formulation of any one of claims 1 to 4, **characterized in that** a weight percentage of the polymer is from 2.5% to 20% based on 100% by weight percentage of the in situ gel formulation.

6. The in situ gel formulation of any one of claims 1 to 5, **characterized in that** a molar ratio of lactic acid to glycolic acid in the polylactic-co-glycolic acid is from 50:50 to 99:1.

7. The in situ gel formulation of any one of claims 1 to 6, **characterized in that** a weight percentage of the lipid is from 17.5% to 60% based on 100% by weight percentage of the in situ gel formulation.

8. The in situ gel formulation of any one of claims 1 to 7, **characterized in that** a weight percentage of the lipid is from 55% to 95% based on 100% by total weight percentage of the polymer and the lipid.

9. The in situ gel formulation of any one of claims 1 to 8, **characterized in that** a weight percentage of the polymer and the lipid is from 25% to 65% based on 100% by weight percentage of the in situ gel formulation.

10. The in situ gel formulation of any one of claims 1 to 9, **characterized in that** the in situ gel formulation further comprises a surfactant having a hydrophilic-lipophilic balance value ranging from 15 to 20, and a weight percentage of the surfactant is from 2.5% to 10% based on 100% by weight percentage of the in situ gel formulation.

11. A preparation method of an in situ gel formulation, **characterized by** comprising:
mixing an active ingredient, polymer and a first solvent to form a first mixture;
mixing lipid and a second solvent to form a second mixture; and
mixing the first mixture and the second mixture to form an in situ gel formulation.

12. The preparation method of claim 11, **characterized in that** the mixing the active ingredient, the polymer and the first solvent comprises:
mixing the active ingredient and the first solvent to form a pre-mixture; and
mixing the pre-mixture and the polymer to form the first mixture.

13. The preparation method of any one of claims 11 to 12, **characterized in that** the mixing the lipid and the second solvent comprises mixing the lipid, the second solvent and a surfactant.

14. A preparation method of an in situ gel formulation, **characterized by** comprising:
mixing polymer and a solvent to form a polymer mixture;
mixing an active ingredient and lipid to form a lipid mixture; and
mixing the polymer mixture and the lipid mixture to form an in situ gel formulation.

15. The preparation method of claim 14, **characterized in that** the mixing the polymer and the solvent comprises mixing the polymer, the solvent and a surfactant.
